# EUROPEAN PATENT APPLICATION

(11) **EP 4 567 027 A1**
(43) Date of publication of application: **11.06.2025**
(21) Application number: 23849396.9
(22) Date of filing: 01.08.2023
(51) Int. Cl.: C07D 233/54, C07C 309/73, C07C 69/608, C07C 69/007

(54) **INTERMEDIATE OF BICYCLIC INHIBITOR AND PREPARATION METHOD THEREFOR**

(30) Priority: 01.08.2022 CN 202210917652
(71) Applicant: Jiangsu Hansoh Pharmaceutical Group Co., Ltd., Lianyungang, Jiangsu 222047 (CN); Shanghai Hansoh Biomedical Co., Ltd., Shanghai 201203 (CN)
(72) Inventor: SUN, Changliang, Shanghai 201203 (CN); ZHANG, Junchi, Shanghai 201203 (CN)
(74) Representative: Murgitroyd & Company
(86) International application number: PCT/CN2023/110505
(87) International publication number: WO 2024/027690

(57) **Abstract**

The present invention provides an intermediate of a bicyclic inhibitor and a preparation method therefor. The present invention particularly relates to an intermediate as shown in formula (VI) and a preparation method therefor. The reaction method uses initial raw materials that are cheap and easy to obtain. The synthesis process is mature, the product quality is stable, the yield and purity are high, the key reagents can be recycled, the production cost is reduced, and the method is suitable for large-scale industrial production and application.

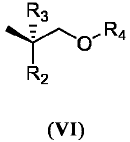

## Description

### Technical Field

The present invention belongs to the field of drug synthesis, and specifically relates to an intermediate of a bicyclic inhibitor and a preparation method therefor.

### Background Art

RET (rearranged during transfection) protein, which is encoded by proto-oncogene RET located on chromosome 10, is a receptor tyrosine kinase composed of an extracellular region, a transmembrane region, and an intracellular kinase region. Ligands of RET are glial cell-derived neurotrophic factor (GDNF) family ligands (GFLs), such as GDN, neurturin (NRTN), artemin (ARTN), and persephin (PSPN). Activation of the receptor also requires the co-action of the co-receptor GFR a family. GFLs form dimers with GFR a, which bind to RET and enable the RET recruited to a cholesterol-rich membrane region. The RET proteins dimerize and undergo autophosphorylation, thereby activating downstream signaling pathways such as RAS-MAPK, PI3K-AKT, and PKC. RET plays an important role in the development of kidney and intestinal nervous system during embryonic development. In addition, RET is also important for the homeostasis of neuroendocrine, hematopoiesis, male germ cells, and other tissues.

Dysfunction of RET protein leads to many diseases. Loss of RET protein function during development leads to a series of congenital diseases, such as Hirschsprung's disease (HSCR) and congenital anomalies of the kidney and urinary tract (CAKUT). In addition, activation mutations in RET protein, including point mutations and RET protein fusion caused by chromosome rearrangement, are also related to the occurrence of many diseases. RET fusion mainly occurs in 1-2% of patients with non-small cell lung cancer (NSCLC) and 5-10% of papillary thyroid cancer, while RET mutations mainly occur in 60% of medullary thyroid cancer. In addition, activation mutations in RET protein have been found in many other tumors, such as breast cancer, gastric cancer, intestinal cancer, and chronic myelomonocytic leukemia.

Although there is a great clinical demand, current treatments against the RET target still have great limitations. Unlike targeted drugs such as ALK and EGFR, they have achieved excellent efficacy. There is currently still no approved targeted drug against the RET target. At present, multi-kinase inhibitors (MKIs), such as vandetanib and cabozantinib, are mostly used in clinical medicine. These multi-kinase inhibitors have the disadvantages of poor selectivity, high side effects, and poor efficacy; furthermore, they cannot overcome drug resistance problems during treatment.

At present, there is no specific targeted drug against the RET target, and there is a great clinical demand. RET inhibitors with a higher selectivity, a better activity, a better safety, and the ability to overcome drug resistance mutations have the potential to treat a variety of cancers and have broad market prospects. In 2015, Jiangsu Hansoh Pharmaceutical Group Co., Ltd. disclosed a series of bicyclic derivatives in patent WO 2020228756 A1, which showed outstanding pharmacological properties and had a strong inhibitory effect on RET kinase activity, but a relatively poor inhibitory effect on KDR kinase activity. The inhibitory effect on KDR/RET kinase activity is highly selective. At present, they have entered the clinical phase II stage of research. However, the following intermediate is used during the preparation of this compound:

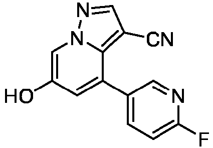

Patent WO 2018071454 A has disclosed a series of RET inhibitors and discloses the key intermediate as shown above for preparing the RET inhibitors and a preparation method therefor. However, the preparation method for this intermediate in the patent is complex, has lengthy reaction steps, with a total of 10 steps, and a relatively low overall yield of the product, and is not suitable for industrial production. Therefore, the present invention is devoted to studying a method for preparing a bicyclic compound that is suitable for industrial production.

### Summary of the Invention

In order to solve the problems existing in the prior art, the inventors have developed a novel method for preparing a RET inhibitor during the long-term research and development process. Through specific intermediates of formulas (V) and (VI) and preparation methods therefor, the present invention can improve the yield and purity of the RET inhibitor compound, which is more conducive to industrial production.

In a first aspect, an object of the present invention is to provide a compound represented by general formula (VI) or a stereoisomer and salt thereof, wherein 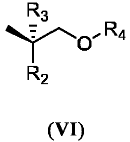
R₂ is selected from hydrogen, deuterium, hydroxyl, amino, C₁₋₃ alkyl, C₁₋₃ deuteroalkyl, C₁₋₃ haloalkyl, C₁₋₃ alkoxy, halo C₁₋₃ alkoxy, or C₁₋₃ hydroxyalkyl, optionally substituted with one or more substituents selected from deuterium, halogen, amino, hydroxyl, cyano, nitro, C₁₋₃ alkyl, C₂₋₄ group, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl;
R₃ is selected from C₁₋₃ alkyl, C₂₋₃ alkenyl, or C₂₋₃ alkynyl, optionally substituted with one or more substituents selected from deuterium, halogen, amino, hydroxyl, cyano, nitro, C₁₋₃ alkyl, C₂₋₄ group, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl;
R₄ is selected from -C(O)(CRₐₐR_{bb})ₙRₐ or -(CRₐₐR_{bb})ₘR_{b};
Rₐₐ or R_{bb} is each independently selected from hydrogen, deuterium, halogen, hydroxyl, amino, or C₁₋₃ alkyl, optionally substituted with one or more substituents selected from deuterium, halogen, amino, hydroxyl, cyano, nitro, C₁₋₃ alkyl, C₂₋₄ group, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl;
Rₐ is selected from C₆₋₁₀ aryl or a 6- to 10-membered heteroaryl containing 1-3 N, O, or S atoms, wherein the C₆₋₁₀ aryl or the 6- to 10-membered heteroaryl containing 1-3 N, O, or S atoms are optionally substituted with one or more substituents selected from deuterium, halogen, amino, hydroxyl, cyano, nitro, C₁₋₃ alkyl, C₂₋₄ group, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl;
R_{b} is selected from hydroxyl protecting groups, preferably substituted or unsubstituted sulfonyl;
m is 0, 1, or 2; and
n is 0, 1, or 2.

In a preferred embodiment of the present invention, R₂ is selected from hydrogen, deuterium, amino, hydroxyl, cyano, nitro, fluorine, chlorine, bromine, methyl, ethyl, isopropyl, methoxy, ethoxy, propoxy, dimethoxymethyl, dimethoxyethyl, fluoromethyl, fluoroethyl, difluoromethyl, difluoroethyl, trifluoromethyl, or trifluoroethyl;
R₃ is selected from methyl, ethyl, propyl, vinyl, allyl, propenyl, ethynyl, propynyl, or propargyl;
R₄ is selected from -C(O)(CRₐₐR_{bb})ₙRₐ or -(CRₐₐR_{bb})ₘR_{b};
Rₐₐ or R_{bb} is each independently selected from hydrogen, deuterium, fluorine, chlorine, hydroxyl, amino, methyl, ethyl, propyl, or isopropyl;
Rₐ is selected from phenyl, naphthyl, pyridine, pyrimidine, or a six-membered ring fused six-membered heteroaryl containing 1-3 N, O, or S atoms, wherein the phenyl, naphthyl, pyridine, pyrimidine, or six-membered ring fused six-membered heteroaryl containing 1-3 N, O, or S atoms is optionally substituted with one or more substituents selected from deuterium, amino, hydroxyl, cyano, nitro, fluorine, chlorine, bromine, methyl, ethyl, isopropyl, methoxy, ethoxy, fluoromethyl, fluoroethyl, difluoromethyl, difluoroethyl, trifluoromethyl, or trifluoroethyl;
R_{b} is selected from benzenesulfonyl, p-toluenesulfonyl, p-nitrobenzenesulfonyl, p-methoxybenzenesulfonyl, p-trifluoromethylbenzenesulfonyl, p-chlorobenzenesulfonyl, or 2-naphthalenesulfonyl, preferably p-nitrobenzenesulfonyl or 2-naphthalenesulfonyl;
m is 0, 1, or 2; and
n is 0, 1, or 2.

In a further preferred embodiment of the present invention,
R₄ is selected from benzenesulfonyl, p-toluenesulfonyl, p-nitrobenzenesulfonyl, p-methoxybenzenesulfonyl, p-trifluoromethylbenzenesulfonyl, p-chlorobenzenesulfonyl, or 2-naphthalenesulfonyl, preferably p-nitrobenzenesulfonyl or 2-naphthalenesulfonyl.

In a further preferred embodiment of the present invention, the compound of formula (VI) is further as represented by general formula (XVII): 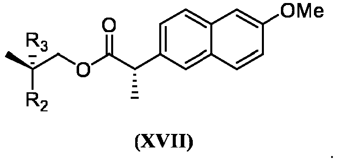 R₂ and R₃ are as represented by general formula (VI).

In another aspect, an object of the present invention is to provide a compound represented by general formula (IX) or a stereoisomer and salt thereof, wherein 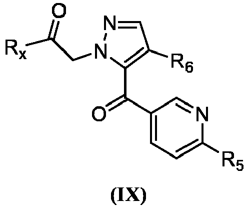
Rₓ is selected from hydrogen, deuterium, C₁₋₃ alkyl, halogen, C₁₋₃ deuteroalkyl, C₁₋₃ haloalkyl, C₁₋₃ alkoxy, halo C₁₋₃ alkoxy, or C₁₋₃ hydroxyalkyl, optionally substituted with one or more substituents selected from deuterium, halogen, amino, hydroxyl, cyano, nitro, C₁₋₃ alkyl, C₂₋₄ group, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl; preferably, R₄ is selected from hydrogen, deuterium, methyl, ethyl, n-propyl, isopropyl, fluorine, chlorine, or bromine;
R₅ is selected from hydrogen, deuterium, C₁₋₃ alkyl, halogen, C₁₋₃ deuteroalkyl, C₁₋₃ haloalkyl, C₁₋₃ alkoxy, halo C₁₋₃ alkoxy, or C₁₋₃ hydroxyalkyl, optionally substituted with one or more substituents selected from deuterium, halogen, amino, hydroxyl, cyano, nitro, C₁₋₃ alkyl, C₂₋₄ group, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl; preferably, R₅ is selected from hydrogen, deuterium, methyl, ethyl, n-propyl, isopropyl, fluorine, chlorine, or bromine; and
R₆ is selected from hydrogen, deuterium, C₁₋₃ alkyl, C₂₋₃ alkenyl, C₂₋₃ alkynyl halogen, cyano, amino, or hydroxyl, preferably hydrogen, deuterium, methyl, ethyl, n-propyl, isopropyl, fluorine, chlorine, bromine, cyano, vinyl, ethynyl, amino, or hydroxyl.

In a further preferred embodiment of the present invention, compounds with the following structures or stereoisomers and salts thereof are provided: 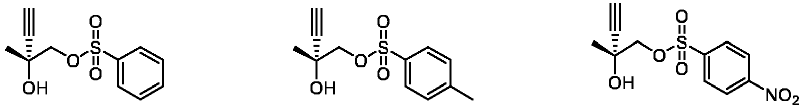 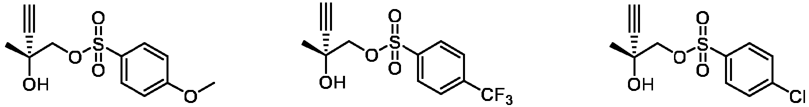 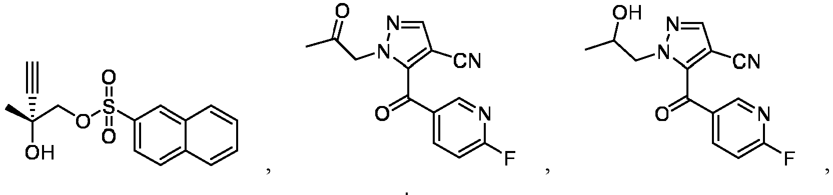 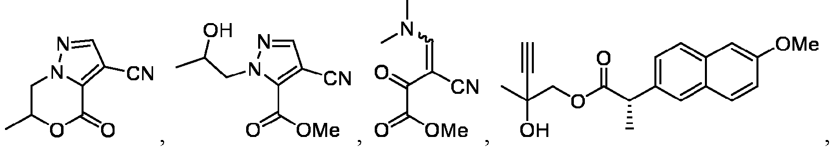 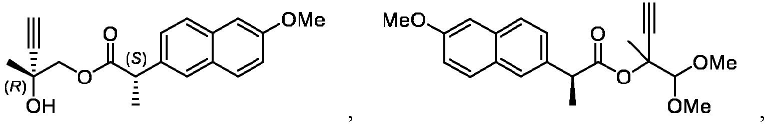 and

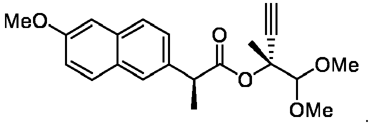

An intermediate for forming the compound of formula (IX) or the stereoisomer and salt thereof is as represented by general formula (X): 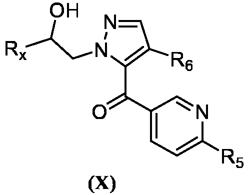 preferably

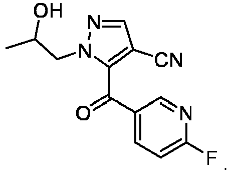

An intermediate for forming the compound of formula (IX) or the stereoisomer and salt thereof is as represented by general formula (XI): 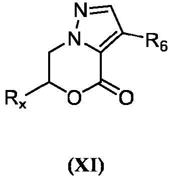 preferably

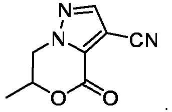

Another object of the present invention is to provide a method for preparing a compound represented by formula (X), comprising the following step: 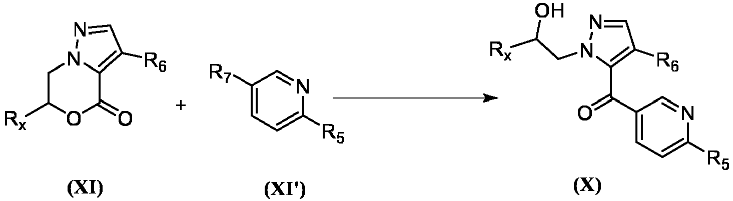 reacting formula **(XI)** with formula **(XI')** to obtain formula **(X),** wherein
Rₓ is selected from hydrogen, deuterium, C1-3 alkyl, or halogen, preferably hydrogen, deuterium, methyl, ethyl, n-propyl, isopropyl, fluorine, chlorine, or bromine;
R₅ is selected from hydrogen, deuterium, C1-3 alkyl, or halogen, preferably hydrogen, deuterium, methyl, ethyl, n-propyl, isopropyl, fluorine, chlorine, or bromine;
R₆ is selected from hydrogen, deuterium, C1-3 alkyl, halogen, cyano, amino, or hydroxyl, preferably hydrogen, deuterium, methyl, ethyl, n-propyl, isopropyl, fluorine, chlorine, bromine, cyano, amino, or hydroxyl;
R₇ is selected from halogen, preferably fluorine, chlorine, or bromine;
preferably, the reaction is carried out in the presence of an organometallic reagent that is selected from an organolithium reagent or an organomagnesium reagent, preferably n-butyl lithium, sec-butyl lithium, isobutyl lithium, tert-butyl lithium, isopropyl magnesium chloride, isopropyl magnesium bromide, or isopropyl magnesium chloride-lithium chloride, more preferably n-butyl lithium; and
preferably, a solvent used in the reaction is selected from one or more of toluene, xylene, acetonitrile, 1,4-dioxane, tetrahydrofuran, 2-methyltetrahydrofuran, ethylene glycol dimethyl ether, or methyl tert-butyl ether, preferably a mixed solvent of tetrahydrofuran and toluene or ethylene glycol dimethyl ether and methyl tert-butyl ether, more preferably tetrahydrofuran and toluene at a volume ratio of 4 : 1-1 : 4 or ethylene glycol dimethyl ether and methyl tert-butyl ether at a volume ratio of 1 : 5-1 : 1, further preferably ethylene glycol dimethyl ether and methyl tert-butyl ether at a volume ratio of 1 : 2 or tetrahydrofuran and toluene at a volume ratio of 1.5 : 1.

In a further preferred embodiment of the present invention, the method for preparing the compound represented by formula **(X)** is as follows:

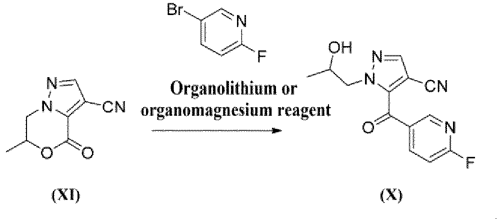

In a further preferred embodiment of the present invention, formula **(XII)** is reacted to obtain formula **(XI),** 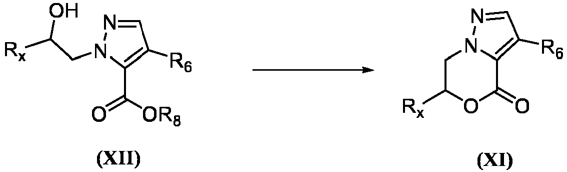
wherein R₈ is C₁₋₃ alkyl, preferably methyl, ethyl, n-propyl, or isopropyl;
preferably, the reaction is carried out in the presence of an acid selected from formic acid, acetic acid, propionic acid, trifluoroacetic acid, benzoic acid, methanesulfonic acid, p-methylbenzenesulfonic acid, phosphoric acid, citric acid, malic acid, or tartaric acid, preferably p-methylbenzenesulfonic acid; and
preferably, the solvent used in the reaction is selected from dichloromethane, chloroform, toluene, chlorobenzene, acetonitrile, 1,4-dioxane, tetrahydrofuran, 2-methyltetrahydrofuran, ethylene glycol dimethyl ether, methyl tert-butyl ether, methanol, N,N-dimethylformamide, N,N-dimethylacetamide, or dimethyl sulfoxide, preferably toluene.

In a further preferred embodiment of the present invention, the method for preparing the compound of formula **(XI)** is as follows:

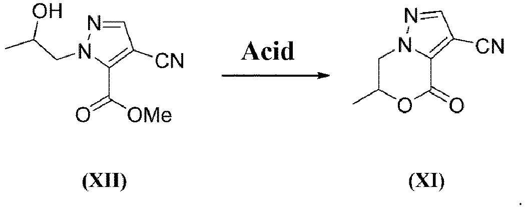

In a further preferred embodiment of the present invention, formula **(XIII)** is reacted with formula **(XIV)** to obtain formula **(XII),** wherein 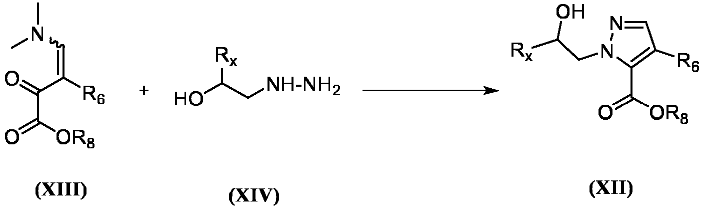
preferably, the reaction is carried out in the presence of an acid selected from formic acid, acetic acid, propionic acid, trifluoroacetic acid, benzoic acid, methanesulfonic acid, p-methylbenzenesulfonic acid, phosphoric acid, citric acid, malic acid, or tartaric acid, preferably acetic acid; and
preferably, the solvent used in the reaction is selected from dichloromethane, chloroform, toluene, chlorobenzene, acetonitrile, 1,4-dioxane, tetrahydrofuran, 2-methyltetrahydrofuran, ethylene glycol dimethyl ether, methyl tert-butyl ether, methanol, N,N-dimethylformamide, N,N-dimethylacetamide, or dimethyl sulfoxide, preferably dichloromethane.

In a further preferred embodiment of the present invention, the method for preparing the compound represented by formula **(XII)** is as follows:

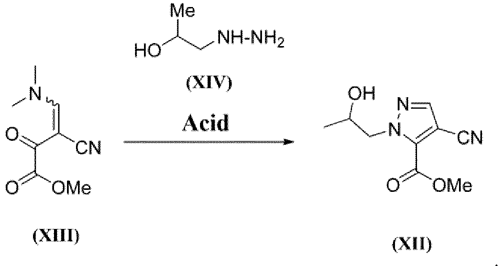

In a further preferred embodiment of the present invention, formula **(XV)** is reacted with N,N-dimethylformamide dimethylacetal under the action of an acid to obtain formula **(XIII),** 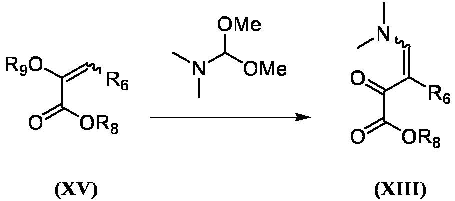
wherein R₉ is selected from Na, K, or H;
preferably, the acid is selected from formic acid, acetic acid, propionic acid, trifluoroacetic acid, benzoic acid, methanesulfonic acid, p-methylbenzenesulfonic acid, phosphoric acid, citric acid, malic acid, or tartaric acid, preferably acetic acid; and
preferably, the solvent used in the reaction is selected from dichloromethane, chloroform, toluene, chlorobenzene, acetonitrile, 1,4-dioxane, tetrahydrofuran, 2-methyltetrahydrofuran, ethylene glycol dimethyl ether, methyl tert-butyl ether, methanol, N,N-dimethylformamide, N,N-dimethylacetamide, or dimethyl sulfoxide, preferably dichloromethane.

In a further preferred embodiment of the present invention, the method for preparing the compound of formula **(XIII)** is as follows:

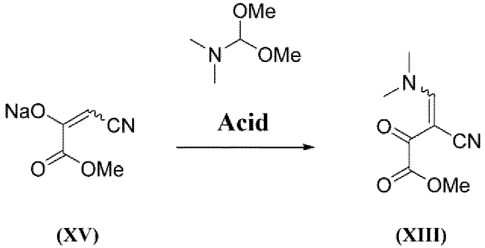

In a further preferred embodiment of the present invention, formula (X) is reacted to obtain formula (IX), wherein 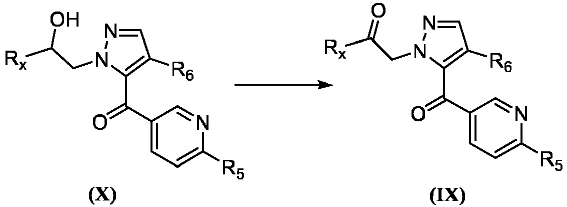
preferably, the reaction is carried out in the presence of an oxidant selected from one or more of sodium hypochlorite, calcium hypochlorite, Dess-Martin periodinane, 2-iodoxybenzoic acid, iodobenzene diacetate, an oxalyl chloride-dimethyl sulfoxide combination reagent, pyridine-sulfur trioxide, 2,2,6,6-tetramethylpiperidine oxide, pyridine-N-oxide, or trichloroisocyanuric acid, preferably a mixed oxidant of 2,2,6,6-tetramethylpiperidine oxide and trichloroisocyanuric acid, more preferably 2,2,6,6-tetramethylpiperidine oxide and trichloroisocyanuric acid at a molar ratio of 1 : 4-1 : 40, further preferably 2,2,6,6-tetramethylpiperidine oxide and trichloroisocyanuric acid at a molar ratio of 1 : 10; and
preferably, the solvent used in the reaction is selected from dichloromethane, chloroform, toluene, chlorobenzene, acetonitrile, 1,4-dioxane, tetrahydrofuran, 2-methyltetrahydrofuran, ethylene glycol dimethyl ether, methyl tert-butyl ether, methanol, N,N-dimethylformamide, N,N-dimethylacetamide, or dimethyl sulfoxide, preferably dichloromethane.

In a further preferred embodiment of the present invention, the method for preparing the compound represented by formula **(IX)** is as follows:

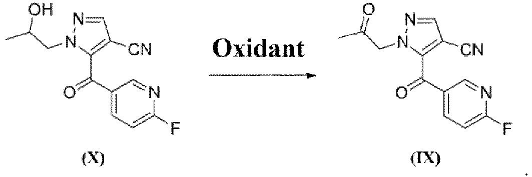

In a further preferred embodiment of the present invention, formula **(IX)** is reacted to obtain formula **(V),** wherein 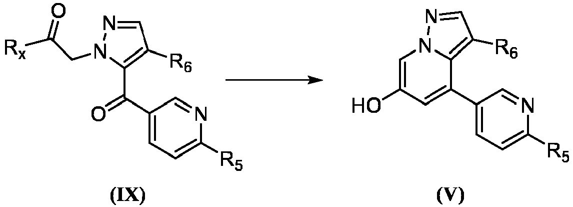
preferably, the reaction is carried out in the presence of an alkali selected from one or more of sodium carbonate, sodium hydroxide, sodium methoxide, sodium ethoxide, sodium isopropoxide, sodium tert-butoxide, sodium hydride, potassium carbonate, potassium hydroxide, potassium methoxide, potassium ethoxide, potassium isopropoxide, potassium tert-butoxide, cesium carbonate, lithium carbonate, lithium hydroxide, lithium chloride, lithium methoxide, lithium ethoxide, lithium isopropoxide, or lithium tert-butoxide, preferably a mixture of lithium hydroxide and lithium chloride, more preferably lithium hydroxide and lithium chloride at a molar ratio of 1 : 4-4 : 1, further preferably lithium hydroxide and lithium chloride at a molar ratio of 1 : 1; and
preferably, a solvent used in the reaction is selected from one or more of toluene, xylene, acetonitrile, 1,4-dioxane, tetrahydrofuran, 2-methyltetrahydrofuran, ethylene glycol dimethyl ether, methyl tert-butyl ether, methanol, ethanol, tert-butanol, water, N,N-dimethylformamide, N,N-dimethylacetamide, or dimethyl sulfoxide, preferably a mixed solvent of ethylene glycol dimethyl ether, methyl tert-butyl ether, and water, preferably ethylene glycol dimethyl ether, methyl tert-butyl ether, and water at a volume ratio of 5-10 : 30-50 : 1, more preferably ethylene glycol dimethyl ether, methyl tert-butyl ether, and water at a volume ratio of 8 : 40 : 1.

In a further preferred embodiment of the present invention, the method for preparing the compound of formula **(V)** is as follows:

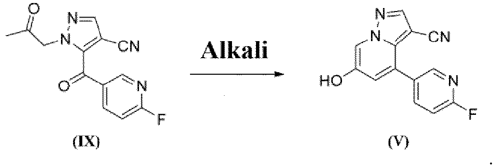

In a further preferred embodiment of the present invention, the method for preparing the compound of formula (V) is as follows:

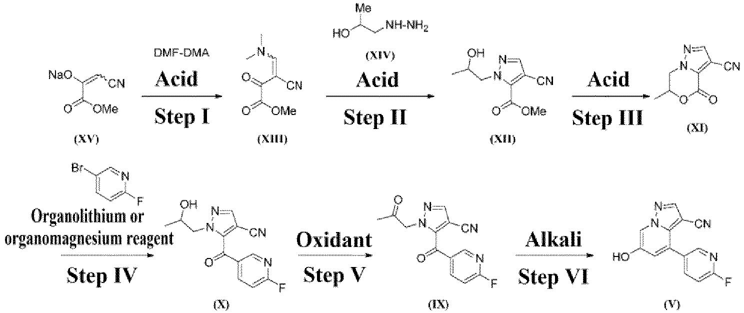

In a further preferred embodiment of the present invention, formula **(XVI)** is reacted with R₄X in the presence of an alkali to obtain formula **(VI),** 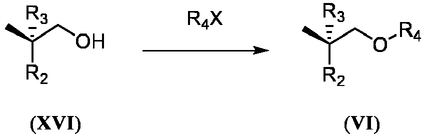
wherein R₂ is selected from hydrogen, deuterium, hydroxyl, amino, or C₁₋₃ alkyl, preferably hydrogen, deuterium, amino, methyl, ethyl, propyl, or isopropyl;
R₃ is selected from C₁₋₃ alkyl, C₁₋₃ alkenyl, or C₁₋₃ alkynyl, preferably methyl, ethyl, propyl, vinyl, allyl, propenyl, ethynyl, propynyl, or propargyl;
R₄ is selected from benzenesulfonyl, p-toluenesulfonyl, p-nitrobenzenesulfonyl, p-methoxybenzenesulfonyl, p-trifluoromethylbenzenesulfonyl, p-chlorobenzenesulfonyl, or 2-naphthalenesulfonyl, preferably p-nitrobenzenesulfonyl or 2-naphthalenesulfonyl;
X is selected from halogen, preferably fluorine, chlorine, or bromine;
preferably, the alkali is selected from pyridine, 2-methylpyridine, 4-methylpyridine, 2,6-dimethylpyridine, 4-dimethylaminopyridine, imidazole, 1-methylimidazole, triethylamine, diisopropylethylamine, 1,8-diazabicyclo[5.4.0]undec-7-ene, triethylenediamine, sodium carbonate, sodium hydroxide, potassium carbonate, potassium hydroxide, cesium carbonate, lithium carbonate, or lithium hydroxide, preferably 2,6-dimethylpyridine or triethylamine; and
preferably, the solvent used in the reaction is selected from dichloromethane, chloroform, toluene, chlorobenzene, acetonitrile, 1,4-dioxane, tetrahydrofuran, 2-methyltetrahydrofuran, ethylene glycol dimethyl ether, methyl tert-butyl ether, N,N-dimethylformamide, N,N-dimethylacetamide, or dimethyl sulfoxide, preferably dichloromethane.

In a further preferred embodiment of the present invention, the method for preparing the compound represented by formula **(VI-1)** is as follows:

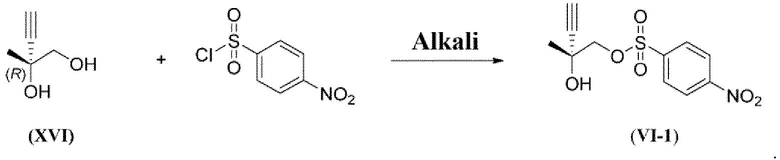

In a further preferred embodiment of the present invention, the method for preparing the compound represented by formula (VI-3) is as follows:

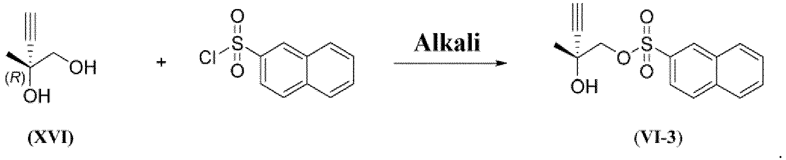

In a further preferred embodiment of the present invention, formula **(XVII)** is reacted with an alkali to obtain formula **(XVI),** wherein 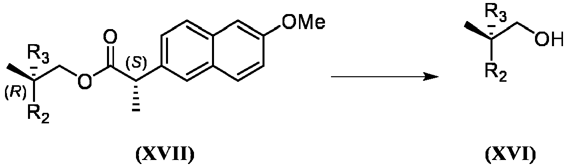
preferably, the reaction is carried out in the presence of an alkali selected from sodium carbonate, sodium hydroxide, sodium methoxide, sodium ethoxide, sodium isopropoxide, sodium tert-butoxide, sodium hydride, potassium carbonate, potassium hydroxide, potassium methoxide, potassium ethoxide, potassium isopropoxide, potassium tert-butoxide, cesium carbonate, lithium carbonate, lithium hydroxide, lithium methoxide, lithium ethoxide, lithium isopropoxide, or lithium tert-butoxide, preferably sodium hydroxide; and
preferably, the solvent used in the reaction is selected from one or more of acetonitrile, 1,4-dioxane, tetrahydrofuran, 2-methyltetrahydrofuran, ethylene glycol dimethyl ether, methyl tert-butyl ether, methanol, ethanol, isopropanol, tert-butanol, ethylene glycol, or water, preferably a mixed solvent of tetrahydrofuran and water. More preferably, the volume ratio of tetrahydrofuran to water is 40 : 1-30 : 1, and further preferably, the volume ratio of tetrahydrofuran to water is 33 : 1.

In a further preferred embodiment of the present invention, the method for preparing the compound represented by formula **(XVI)** is as follows:

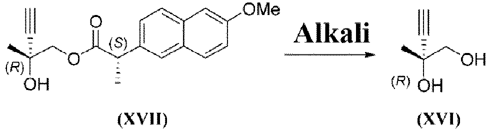

In a further preferred embodiment of the present invention, formula **(XVII-RAC)** is resolved to obtain formula **(XVII),** wherein 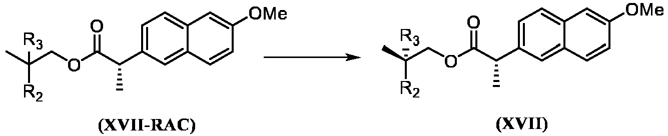 preferably, a resolution solvent is selected from one or more of dichloromethane, chloroform, toluene, chlorobenzene, acetonitrile, acetic acid, 1,4-dioxane, tetrahydrofuran, 2-methyltetrahydrofuran, ethylene glycol dimethyl ether, methyl tert-butyl ether, methanol, N,N-dimethylformamide, N,N-dimethylacetamide, or dimethyl sulfoxide, preferably a combination of toluene and acetonitrile or a combination of toluene and acetic acid, more preferably toluene and acetonitrile solvents used at a volume ratio selected from 20 : 1-3 : 1 or toluene and acetic acid solvents at a volume ratio selected from 20 : 1-5 : 1, further preferably toluene and acetonitrile solvents at a volume ratio selected from 10 : 1-4 : 1 or toluene and acetic acid solvents at a volume ratio selected from 15 : 1-10 : 1, most preferably toluene and acetonitrile solvents at a volume ratio of 4 : 1 or toluene and acetic acid solvents at a volume ratio of 12 : 1 or 12.5 : 1.

In a further preferred embodiment of the present invention, the method for preparing the compound represented by formula (XVII) is as follows:

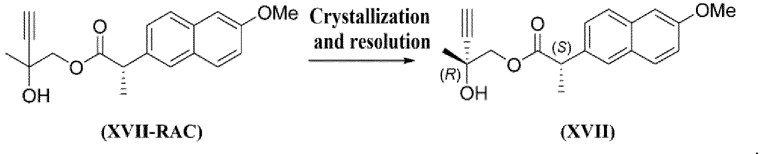

In a further preferred embodiment of the present invention, formula **(XVI-RAC)** is reacted to obtain formula **(XVII-RAC),** wherein 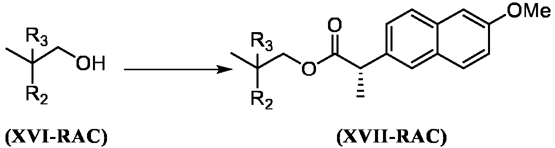
preferably, the reaction is carried out in the presence of a naproxen chlorinating reagent selected from oxalyl chloride, thionyl dichloride, phosphorus oxychloride, phosphorus pentachloride, or triphosgene, preferably oxalyl chloride and triphosgene;
preferably, the alkali is selected from pyridine, 2-methylpyridine, 4-methylpyridine, 2,6-dimethylpyridine, 4-dimethylaminopyridine, imidazole, 1-methylimidazole, triethylamine, diisopropylethylamine, 1,8-diazabicyclo[5.4.0]undec-7-ene, triethylenediamine, sodium carbonate, sodium hydroxide, potassium carbonate, potassium hydroxide, cesium carbonate, lithium carbonate, or lithium hydroxide, preferably 2,6-dimethylpyridine; and
preferably, the solvent used in the reaction is selected from dichloromethane, chloroform, toluene, chlorobenzene, acetonitrile, 1,4-dioxane, tetrahydrofuran, 2-methyltetrahydrofuran, ethylene glycol dimethyl ether, methyl tert-butyl ether, N,N-dimethylformamide, N,N-dimethylacetamide, or dimethyl sulfoxide, preferably dichloromethane.

In a further preferred embodiment of the present invention, the method for preparing the compound represented by formula **(XVII-RAC)** is as follows:

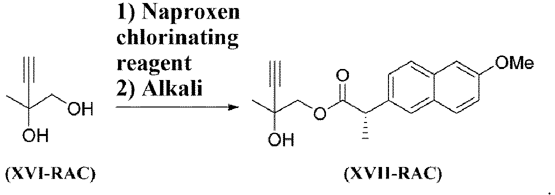

In a further preferred embodiment of the present invention, formula (XVIII) is reacted with a reducing agent to obtain formula (XVI), wherein 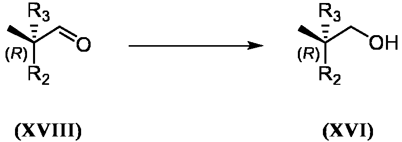
preferably, the reducing agent is selected from sodium borohydride, potassium borohydride, sodium triacetoxyborohydride, sodium cyanoborohydride, borane tetrahydrofuran complex, sodium trimethoxyborohydride, or lithium aluminum hydride, preferably sodium triacetoxyborohydride.
Preferably, the reaction solvent is selected from dichloromethane, chloroform, toluene, chlorobenzene, acetonitrile, 1,4-dioxane, tetrahydrofuran, 2-methyltetrahydrofuran, ethylene glycol dimethyl ether, methanol, ethanol, isopropanol, tert-butanol, or ethylene glycol, preferably a mixed solvent of dichloromethane and methanol, more preferably dichloromethane and methanol at a ratio of 5-20 : 1.

In a further preferred embodiment of the present invention, the method for preparing the compound represented by formula **(XVI)** is as follows:

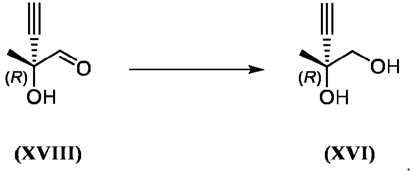

In a further preferred embodiment of the present invention, formula **(XIX)** is reacted in the presence of an acid to obtain formula **(XVIII),** 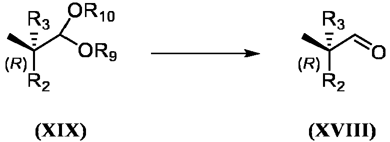
wherein R₉ and R₁₀ are each independently C₁₋₃ alkyl, preferably methyl, ethyl, n-propyl, or isopropyl;
preferably, the acid is selected from concentrated hydrochloric acid, concentrated sulfuric acid, concentrated phosphoric acid, a solution of hydrogen chloride in 1,4-dioxane, trifluoroacetic acid, trifluoromethanesulfonic acid, benzenesulfonic acid, methanesulfonic acid, or p-methylbenzenesulfonic acid, preferably trifluoroacetic acid; and
preferably, the solvent used in the reaction is selected from N,N-dimethylformamide, N,N-dimethylacetamide, dimethyl sulfoxide, N-methylpyrrolidone, dichloromethane, chloroform, toluene, chlorobenzene, acetonitrile, 1,4-dioxane, tetrahydrofuran, 2-methyltetrahydrofuran, ethylene glycol dimethyl ether, methanol, ethanol, isopropanol, tert-butanol, ethylene glycol, or water, preferably dichloromethane.

In a further preferred embodiment of the present invention, the method for preparing the compound represented by formula **(XVIII)** is as follows:

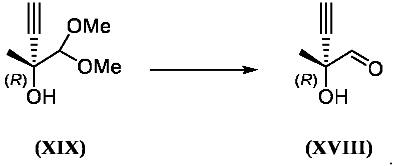

In a further preferred embodiment of the present invention, formula (XX) is reacted in the presence of an alkali to obtain formula (XIX), wherein 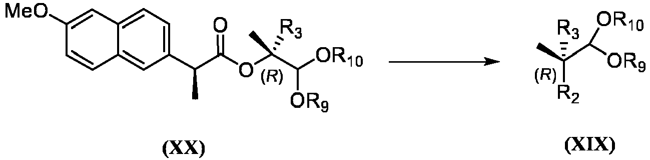
preferably, the alkali is selected from sodium carbonate, sodium hydroxide, sodium methoxide, sodium ethoxide, sodium isopropoxide, sodium tert-butoxide, sodium hydride, potassium carbonate, potassium hydroxide, potassium methoxide, potassium ethoxide, potassium isopropoxide, potassium tert-butoxide, cesium carbonate, lithium carbonate, lithium hydroxide, lithium methoxide, lithium ethoxide, lithium isopropoxide, or lithium tert-butoxide, preferably sodium hydroxide; and
preferably, the solvent used in the reaction is selected from one or more of acetonitrile, 1,4-dioxane, tetrahydrofuran, 2-methyltetrahydrofuran, ethylene glycol dimethyl ether, methyl tert-butyl ether, methanol, ethanol, isopropanol, tert-butanol, ethylene glycol, or water, preferably a mixed solvent of tetrahydrofuran and water, more preferably tetrahydrofuran and water at a volume ratio of 20 : 1-5 : 1, further preferably tetrahydrofuran and water at a volume ratio of 10 : 1.

In a further preferred embodiment of the present invention, the method for preparing the compound represented by formula **(XIX)** is as follows:

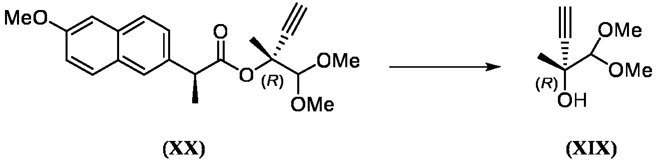

In a further preferred embodiment of the present invention, formula **(XX-RAC)** is resolved to obtain formula **(XX),** wherein 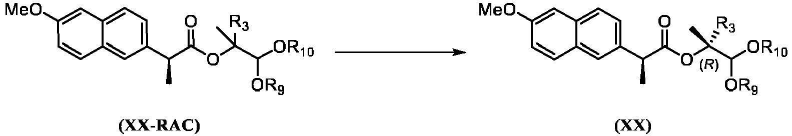 preferably, the resolution solvent is selected from one or more of dichloromethane, chloroform, toluene, chlorobenzene, acetonitrile, 1,4-dioxane, tetrahydrofuran, acetone, ethylene glycol dimethyl ether, methyl tert-butyl ether, ethyl acetate, isopropyl acetate, n-heptane, methanol, ethanol, isopropanol, n-butanol, and water, preferably isopropanol.

In a further preferred embodiment of the present invention, the method for preparing the compound represented by formula **(XX)** is as follows:

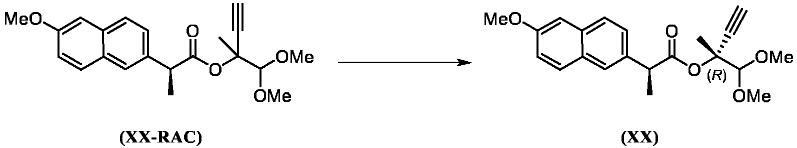

In a further preferred embodiment of the present invention, formula **(XIX-RAC)** is reacted with a naproxen chlorinating reagent under the action of an alkali to obtain formula **(XX-RAC),** wherein 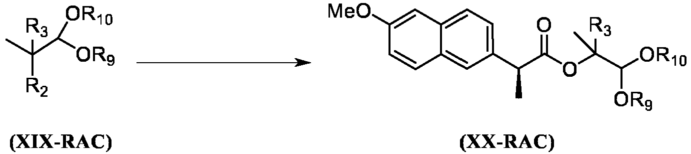
preferably, the chlorinating reagent is selected from oxalyl chloride, thionyl dichloride, phosphorus oxychloride, phosphorus pentachloride, or triphosgene, preferably oxalyl chloride and triphosgene;
preferably, the alkali is selected from pyridine, 2-methylpyridine, 4-methylpyridine, 2,6-dimethylpyridine, 4-dimethylaminopyridine, imidazole, 1-methylimidazole, triethylamine, diisopropylethylamine, 1,8-diazabicyclo[5.4.0]undec-7-ene, triethylenediamine, sodium carbonate, sodium hydroxide, potassium carbonate, potassium hydroxide, cesium carbonate, lithium carbonate, or lithium hydroxide, preferably pyridine; and
preferably, the solvent used in the reaction is selected from dichloromethane, chloroform, toluene, chlorobenzene, acetonitrile, 1,4-dioxane, tetrahydrofuran, 2-methyltetrahydrofuran, ethylene glycol dimethyl ether, methyl tert-butyl ether, N,N-dimethylformamide, N,N-dimethylacetamide, or dimethyl sulfoxide, preferably dichloromethane.

In a further preferred embodiment of the present invention, the method for preparing the compound represented by formula **(XX-RAC)** is as follows:

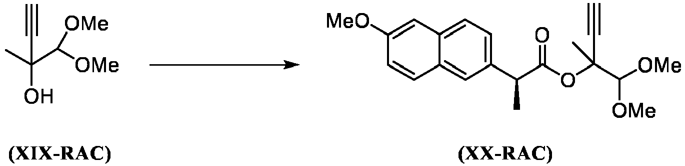

In a further preferred embodiment of the present invention, the method for preparing the compound of formula **(VI)** is as follows:

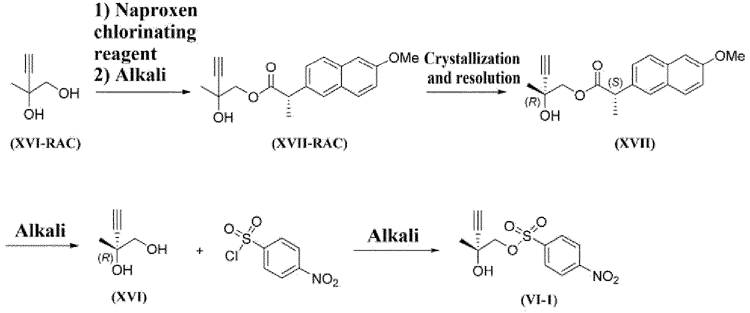

In a further preferred embodiment of the present invention, the method for preparing the compound of formula **(VI)** is as follows:

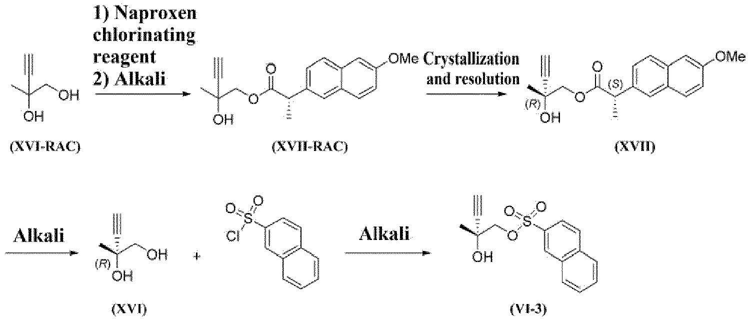

In a further preferred embodiment of the present invention, the method for preparing the compound of formula **(VI)** is as follows:

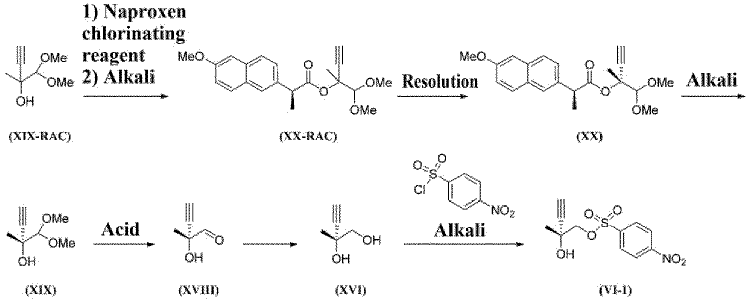

In a further preferred embodiment of the present invention, the method for preparing the compound of formula **(VI)** is as follows:

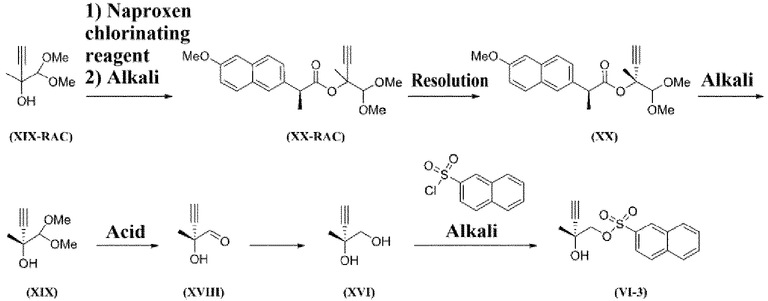

The method for preparing the compound represented by formula **(VI)** according to the present invention uses starting raw materials that are cheap and easy to obtain in the reaction method. The synthesis process is mature, the product quality is stable, the key reagents can be recycled, the production cost is reduced, and the method is suitable for large-scale industrial production and application.

In the present invention, formula (V) is prepared by a brand-new preparation method. The reaction method uses starting raw materials that are cheap and easy to obtain. The synthesis process is mature, tandem reaction can be performed during reaction, shortening reaction steps, the operation is simple, a column chromatography purification process is avoided, the product quality is stable, the production cost is reduced, and the yield and purity are improved. The method is very suitable for large-scale industrial production and application.

Through specific intermediates of formulas (V) and (VI) and preparation methods therefor, the present invention can improve the yield and purity of the RET inhibitor compound, which is more conducive to industrial production.

### Detailed Description of Embodiments

Detailed description: Unless stated to the contrary, the following terms used in the description and claims have the following meanings.

"Alkyl" refers to linear and branched alkyl groups comprising 1 to 4 carbon atoms, and alkyl refers to a saturated aliphatic hydrocarbon group, such as methyl, ethyl, n-propyl, isopropyl, n-butyl, isobutyl, tert-butyl, and sec-butyl.

The present invention will be further described below in detail and completely with reference to examples. These examples are by no means intended to limit the present invention, and the present invention is not limited to the content of the examples.

The structure of the compound of the present invention is determined by nuclear magnetic resonance (NMR) and/or liquid chromatography-mass spectrometry (LC-MS). NMR chemical shifts are given in parts per million (ppm). NMR measurement is carried out by Bruker AVANCE-400 nuclear magnetic resonance spectrometer. Solvents for measurement are deuterated dimethyl sulfoxide (DMSO-Cl6), deuterated methanol (CD3(10), and deuterated chloroform (CDCl3), and the internal standard is tetramethylsilane (TMS).

Agilent 1200 Infinity Series mass spectrometer is used for measurement by liquid chromatography-mass chromatography (LC-MS). HPLC measurement is carried out by Agilent 1200DAD high-pressure liquid chromatograph (Sunfire C18 150 X 4.6 mm chromatographic column) and Waters 2695-2996 high-pressure liquid chromatograph (Gimini C18 150 x 4.6 mm chromatographic column).

Yantai Huanghai HSGF254 or Qingdao GF254 silica gel plate is used as a thin layer chromatography silica gel plate, with the specification for TLC being 0.15-0.20 mm and the specification for product separation and purification by thin layer chromatography being 0.4-0.5 mm. Column chromatography generally involves using Yantai Huanghai silica gel 200-300 mesh silica gel as a carrier.

Unless otherwise specified, all the reactions in the present invention are under continuous magnetic stirring.

### Example 1

### Preparation of the compound represented by formula (XIII)

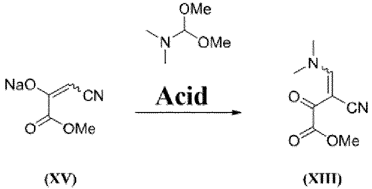

The compound represented by formula **(XV)** (140 g, 0.942 mol), N,N-dimethylformamide dimethylacetal (337 g, 2.83 mol), and dichloromethane (700 mL) were successively added to a three-necked flask, and the mixture was cooled to 0-2°C and uniformly stirred. A solution of acetic acid (170 g, 2.83 mol) in dichloromethane (140 mL) was slowly added under stirring at 0-2°C, and the mixture was heated to 20-25°C and reacted under stirring for 3 hours. The reaction was complete as determined by HPLC analysis. The reaction was quenched by slowly adding a saturated sodium bicarbonate aqueous solution (700 mL) to the reaction mixture, which was left to stand for liquid separation, and the aqueous phase was extracted three times with dichloromethane (700 mL x 3). The organic phases were combined and then washed with water, the combined organic phases were concentrated and dried in vacuum to obtain a yellow-green solid product, which was dried in vacuum to obtain 150 g of the compound represented by formula **(XIII)** as a product, with a yield of 87.4%.

The above product was directly brought to the next step.

MS, *m*/*z* (ESI): 183.1 [M+H]⁺.

¹H NMR, (400 MHz, CDCl₃) δ 7.93 (s, 1H), 3.87 (s, 3H), 3.48 (s, 3H), 3.33 (s, 3H).

### Example 2

### Preparation of the compound represented by formula (XI)

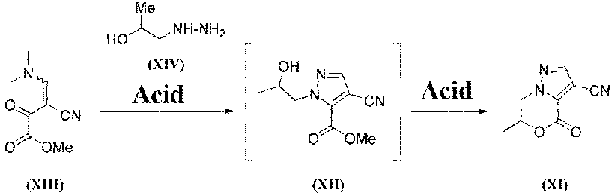

The compound represented by formula **(XIII)** (90.0 g, 494 mmol), as a raw material, was taken and added to a reactor, followed by toluene (360 mL) at room temperature of 20-25°C and then acetic acid (270 mL). The mixture was stirred and dissolved clear. The mixture was cooled to 0-2°C, a solution of the compound represented by formula **(XIV)** (53.5 g, 593 mmol) in dichloromethane (45 mL) was slowly added under stirring, and the reaction was heated to 40-45°C and stirred for 4-6 hours. The reaction was complete as determined by HPLC analysis. The reaction liquid was cooled to room temperature, added to water (900 mL), fully washed, and then left to stand for liquid separation. The aqueous phase was extracted twice with dichloromethane (900 mL x 2), and the organic phases were combined, concentrated, and subjected to solvent displacement with toluene (900 mL x 2) to obtain a toluene solution/mixture of the compound represented by formula **(XII)** as an intermediate product, which was directly brought to the next reaction.

p-Toluenesulfonic acid monohydrate (14.1 g, 74.1 mmol) was added to the mixture obtained in the previous step and heated to an external temperature of 120°C. The reaction was complete as determined by HPLC analysis. After the reaction, the mixture was cooled to room temperature of 20-25°C, and the reaction liquid was slowly added to an ice-water mixture (about 900 mL), whereupon a large amount of a yellow solid precipitated. Ethyl acetate (900 mL x 3) was added for extraction three times, and the obtained organic phases were combined and concentrated. Methyl tert-butyl ether (450 mL) was added, and the mixture was slurried at 50-55°C for 2-3 hours. n-Heptane (270 mL) was slowly dropwise added, and the mixture was slurried at 50-55°C for 1-2 hours, slowly cooled to room temperature, and filtered. The filter cake was fully rinsed with a methyl tert-butyl ether/n-heptane mixture (methyl tert-butyl ether/n-heptane = 2/3, 90 mL). The filter cake was dried in a vacuum drying oven to obtain 42.4 g of a crude product of the compound represented by formula (XI), as a pale yellow powder solid, with a yield of 48.6% of the tandem reaction.

MS, m/z (ESI): 178.1 [M+H]⁺.

¹H NMR, (400 MHz, CDCl₃) δ 7.93 (s, 1H), 5.05-4.96 (m, 1H), 4.58-4.53 (m, 1H), 4.27-4.21 (m, 1H), 1.49 (d, *J* = 8.0 Hz, 3H).

### Example 3

### Preparation of the compound represented by formula (X)

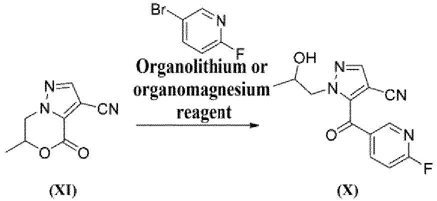

The compound represented by formula **(XI)** (9.30 g, 52 mmol) and 2-fluoro-5-bromopyridine (10.75 g, 60 mmol), as raw materials, were added to a three-necked flask at room temperature of 20-25°C. Under N₂ flow protection, THF (60 mL) and toluene (40 mL) were added to the three-necked flask and uniformly stirred. The reaction was cooled to -60°C to -70°C, and an n-butyl lithium solution (52.8 mL, 2.5 M in hexane) was added. After the addition was complete, the reaction was maintained at -60°C to -70°C for 3 h and slowly heated to -30°C to -25°C, and the reaction was maintained at -30°C to -20°C for 3 hours. The reaction was quenched by adding a trifluoroacetic acid aqueous solution (40 mL, 50%, V/V) at -30°C to - 25°C. After the addition was complete, the reaction product was heated to room temperature of 20-25°C and stirred for 3-4 hours. Water (80 mL) was added to the reaction, and the mixture was uniformly stirred and then left to stand for liquid separation. The upper organic phase was retained. Ethyl acetate (60 mL in total) was added to the aqueous phase for extraction twice, and the organic phases were combined. The combined organic phases were washed once with a saturated sodium bicarbonate aqueous solution (60 mL) and left to stand for liquid separation. The organic phase was retained. The aqueous phase was back-extracted once with ethyl acetate (30 mL). The organic phases were combined. Dichloromethane (100 mL in total) was added for solvent displacement to obtain the compound represented by formula (X) as a crude product, which was directly brought to the next step.

MS, *m*/*z* (ESI): 275.1 [M+H]⁺.

### Example 4

### Preparation of the compound represented by formula (IX)

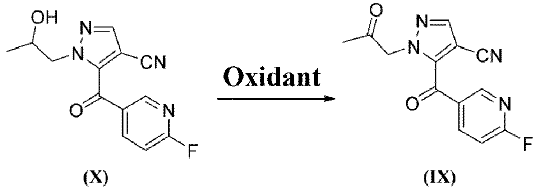

At room temperature of 20-25°C, a solution of the compound represented by formula (X) (32.9 g, 120 mmol) in dichloromethane (about 200 mL) was added to a reactor, and sodium bicarbonate (15.2 g, 180 mmol) and potassium bromide (1.43 g, 12.0 mmol) were added and uniformly stirred at room temperature of 20-25°C. A first batch of 2,2,6,6-tetramethylpiperidine oxide (375 mg, 2.40 mmol) was added under stirring at 20-25°C, and a first batch of trichloroisocyanuric acid (5.58 g, 24.0 mmol) was then slowly added. After the addition was complete, the reaction was carried out at 20-25°C for 2 hours. A second batch of 2,2,6,6-tetramethylpiperidine oxide (375 mg, 2.40 mmol) was further added under stirring at 20-25°C, and trichloroisocyanuric acid (5.58 g, 24.0 mmol) was then slowly added. Then, a third batch, fourth batch, and fifth batch of 2,2,6,6-tetramethylpiperidine oxide (375 mg, 2.40 mmol) and trichloroisocyanuric acid (5.58 g, 24.0 mmol) were further slowly added under stirring at 20-25°C. The reaction was complete as determined by HPLC analysis. The solid was rinsed with dichloromethane (40 mL) and the organic phases were combined. Isopropanol (7.2 g, 120 mmol) was added to the organic phase. The reaction was quenched. The organic phase was washed with a saturated sodium bicarbonate aqueous solution and water, methyl tert-butyl ether was added, and the mixture was concentrated and dried to obtain 24.2 g of the compound represented by formula **(IX),** as a white powdery solid product, with an overall yield of 74.0% over two steps.

MS, *m*/*z* (ESI): 273.1 [M+H]⁺.

¹H NMR (400 MHz, DMSO) δ 8.72 (dd, *J =* 2.1, 1.2 Hz, 1H), 8.50-8.39 (m, 1H), 8.37 (s, 1H), 7.49-7.46 (m, 1H), 5.51 (s, 2H), 2.22 (s, 3H).

### Example 5

### Preparation of the compound represented by formula (V)

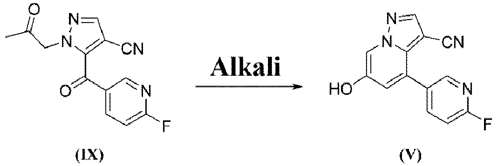

At room temperature of 20-25°C, the compound represented by formula **(IX)** (544 g, 2.0 mol) and lithium chloride (168 g, 4.0 mol) were added to a three-necked flask. Methyl tert-butyl ether (10 L), ethylene glycol dimethyl ether (2.0 L), and water (250 mL) were added. Lithium hydroxide monohydrate (168 g, 4.0 mol) was added to a reaction flask at room temperature of 20-25°C and uniformly stirred, and the reaction was stirred overnight for 16-20 hours at 25-30°C. The reaction was complete as determined by HPLC analysis. At room temperature, aqueous ammonia (20%, 10 L) and ethyl acetate (2 L) were added to the reaction mixture and fully stirred. After liquid separation, the organic phase was discarded, and a citric acid aqueous solution (10-12%, w/w) was slowly added to the aqueous phase under stirring at room temperature to adjust the pH value to 2-3, whereupon a large amount of a solid precipitated. After filtration, a crude solid product was collected and added to a mixed solvent of dimethyl sulfoxide (1.6 L) and water (800 mL), and the mixture was slurried overnight at room temperature. After filtration, a solid was collected to obtain 381 g of the target product, a refined pure product as an off-white solid powder, with a yield of 75%. The purity was 98.1% as measured by an HPLC method.

MS, *m*/*z* (ESI): 255.1 [M+H]⁺.

¹H NMR (400 MHz, DMSO-*d*₆) δ 10.40 (s, 1H), 8.54 (s, 1H), 8.47 (d, *J* = 2.5 Hz, 1H), 8.41 (d, *J =* 1.9 Hz, 1H), 8.24 (td, *J =* 8.1, 2.6 Hz, 1H), 7.37 (dd, *J =* 8.4, 2.7 Hz, 1H), 7.31 (d, *J =* 2.0 Hz, 1H).

### Example 6 Preparation step of naproxen chloride

Naproxen (500 g, 2.17 mol) and dichloromethane (2.0 L) were added to a reactor, followed by N,N-dimethylformamide (12 mL, 7 mol%). The reactor was placed in a water bath at 0°C. A solution of oxalyl chloride (305 g, 2.40 mol) in dichloromethane (1.0 L) was dropwise added to the reaction flask and then maintained at 0°C under stirring for 6-8 hours. The reaction was complete as determined by HPLC analysis. After concentration, 540 g of naproxen acyl chloride as a solid product was obtained with a yield of 99%. The product was directly brought to the next step.

MS, *m*/*z* (ESI): 249.1 [M+H]⁺.

¹H NMR (400 MHz, CDCl₃) δ 7.75-7.66 (m, 3H), 7.32-7.35 (m, 1H), 7.18-7.12 (m, 2H), 4.23 (q, *J =* 8.0 Hz, 1H), 3.90 (s, 3H), 1.65 (d, *J =* 8.0 Hz, 3H).

### Example 7

The compound represented by formula **(XVI)**

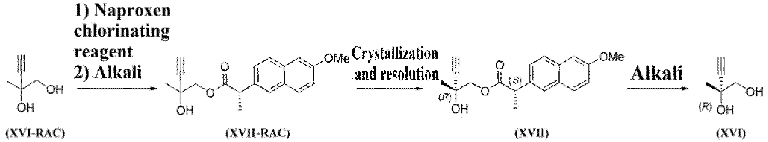

### Step I. Esterification step: Preparation of the compound represented by formula (XVII-RAC)

The compound represented by formula **(XVI-RAC)** (78.5 g, 785 mmol), dichloromethane (400 mL), and 2,6-dimethylpyridine (168 g, 1.57 mol) were added to a reactor, and the reaction was placed in a water bath at 0°C. A solution of the prepared naproxen chloride (214.8 g, 864 mmol) in dichloromethane (800 mL) was dropwise added to the reaction flask and reacted at 0°C for 4 hours. The reaction was complete as determined by GC analysis. The reaction was quenched by adding an acetic acid aqueous solution (240 mL, 5 M), and the reaction product was then washed with a saturated sodium bicarbonate aqueous solution (240 mL x 4). The the organic phases were concentrated. Toluene (240 mL) and n-heptane (880 mL) were added. After filtration, a large amount of an off-white solid was obtained, which was dried to obtain 196 g of the compound represented by formula **(XVII-RAC).** The yield of this step was 80%. The product was a white or off-white solid powder.

MS, *m*/*z* (ESI): 313.1 [M+H]⁺.

¹H NMR (400 MHz, CDCl₃) δ 7.72-7.69 (m, 3H), 7.45-7.41 (m, 1H), 7.16-7.11 (m, 2H), 4.25 (dd, *J =* 16.0, 8.0 Hz, 1H), 4.06-3.94 (m, 2H), 3.91 (s, 3H), 2.36-2.28 (m, 1H), 2.22 (s, 1H), 1.63 (dd, *J =* 8.0, 4.0 Hz, 3H), 1.40 (s, 3H).

### Step II. Crystallization and resolution step: Preparation of the compound represented by formula (XVII)

First resolution: A reaction flask was taken, to which the compound represented by formula **(XVII-RAC)** (196 g, 627 mmol) and toluene (1200 mL) were added. The mixture was heated to 55-60°C and stirred until dissolved clear. After cooling to 30-35°C, the compound represented by formula **(XVII)** (200 mg, 0.001 w/w, *dr* = 95%) was slowly added thereto. After the addition was complete, the mixture was further cooled to 20-25°C and maintained at room temperature for 12-16 hours under stirring. After filtration, a solid was collected, and the filter cake was rinsed with toluene (200 mL) and dried. 122 g of a solid was obtained. The yield of the first resolution was 62%. The *dr* value of the solid product was 72.0%, as measured by chiral column HPLC.

Second resolution: Toluene (850 mL) was added to the solid sample obtained after the first resolution. The mixture was heated to 50-55°C, stirred for 1 hour, cooled to 20-25°C, and stirred for 12-16 hours. After filtration, a solid was collected, and the filter cake was rinsed with toluene (200 mL) and dried to obtain 78.0 g of the compound represented by formula **(XVII)** as a solid. The yield of the second resolution was 64%. The overall yield was 40% over the two steps of resolution. The *dr* value of the solid product was 93.0%, as measured by chiral column HPLC.

Alternatively, the following resolution steps were taken and operated:
First resolution: A reaction flask was taken, to which the compound represented by formula **(XVII-RAC)** (196 g, 627 mmol) was added, followed by toluene (480 mL) and acetonitrile (120 mL). The mixture was heated to 55-60°C and stirred until dissolved clear. After cooling to 37-39°C, the compound represented by formula **(XVII)** (1.0 g, 0.005 w/w, *dr =* 95%) was slowly added thereto as a seed crystal. After the addition was complete, the mixture was maintained at 37-39°C for 1 hour under stirring, then cooled to 22-23°C, and stirred for 11-12 hours. After filtration, a solid was collected, and the filter cake was rinsed with a mixed solvent of toluene (200 mL) and acetonitrile (20 mL) and dried. 72.1 g of a solid was obtained. The yield of the first resolution was 36.8%. The *dr* value of the solid product was 68.2%, as measured by chiral column HPLC.
Second resolution: Toluene (265 mL) and acetic acid (22 mL) were added to the solid sample obtained after the first resolution, heated to 65-70°C, and stirred for 1 hour. After cooling to 58-59°C, the compound represented by formula **(XVII)** (2.0 g, 0.01 w/w, *dr =* 99%) was slowly added thereto as a seed crystal. After the addition was complete, the mixture was maintained at 58-59°C for 2 hours under stirring, then cooled to 24-25°C, and stirred for 10-11 hours. After filtration, a solid was collected, and the filter cake was rinsed with a mixed solvent of toluene (50 mL) and acetonitrile (4 mL) and dried to obtain 53.7 g of the compound represented by formula **(XVII)** as a solid. The yield of the second resolution was 74.5%, and the overall yield was 27% over the two steps of resolution. The *dr* value of the solid product was 95.2%, as measured by chiral column HPLC.
Third resolution: Toluene (192 mL) and acetic acid (16 mL) were added to the solid sample obtained after the second resolution, heated to 65-70°C, and stirred for 1 hour. After cooling to 63-64°C, the compound represented by formula (XVII) (2.0 g, 0.01 w/w, *dr =* 99%) was slowly added thereto as a seed crystal. After the addition was complete, the mixture was maintained at 63-64°C for 2 hours under stirring, then cooled to 24-25°C, and stirred for 7-8 hours. After filtration, a solid was collected, and the filter cake was rinsed with a mixed solvent of toluene (25 mL) and acetonitrile (2 mL) and dried to obtain 48.1 g of the compound represented by formula **(XVII)** as a solid. The yield of the second resolution was 89.6%, and the overall yield was 24.5% over the three steps of resolution. The *dr* value of the solid product was 99.0%, as measured by chiral column HPLC.

MS, *m*/*z* (ESI): 313.1 [M+H]⁺.

¹H NMR (400 MHz, CDCl₃) δ 7.71-7.68 (m, 3H), 7.43-7.41 (m, 1H), 7.15-7.10 (m, 2H), 4.23 (d, *J =* 12.0 Hz, 1H), 4.04 (d, *J* = 8.0 Hz, 1H), 3.97-3.92 (m, 1H), 3.91 (s, 3H), 2.36 (s, 1H), 2.14 (br, 1H), 1.62 (d, *J=* 8.0 Hz, 3H), 1.40 (s, 3H).

### Step III. Hydrolysis step: Preparation of the compound represented by formula (XVI)

A reaction flask was taken, to which the compound represented by formula **(XVII)** (50.0 g, 160 mmol) and a mixed solvent of tetrahydrofuran (500 mL) and water (15 mL) were added and uniformly stirred. Sodium hydroxide solid (12.8 g, 320 mL) was added in batches at 20-25°C and reacted under stirring at 20-25°C for 3 hours. The reaction was complete as determined by HPLC analysis. The reacted mixture was filtered to remove a solid naproxen sodium salt, and the solid filter cake was rinsed with tetrahydrofuran (50 mL). The filtrates were combined. Dichloromethane was added for solvent replacement, and after concentration, 20.2 g of the compound represented by formula **(XVI)** was obtained. The content was 79.1% (w/w), and after conversion, the yield of this step was 99%. The product was directly brought to the next step.

MS, *m*/*z* (ESI): 101.1 [M+H]⁺.

¹H NMR was the same as that reported previously.

### Example 8

### Preparation of the compound represented by formula (VI-1)

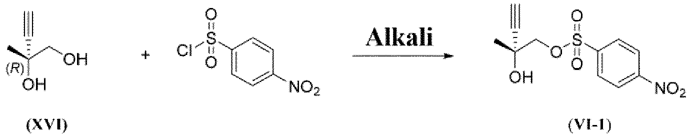

The compound represented by formula **(XVI)** (16.0 g, 160 mmol), dichloromethane (120 mL), and 2,6-dimethylpyridine (34.3 g, 320 mmol) were successively added to a reactor, and the reaction was placed in a water bath at 0°C and stirred until dissolved uniform. p-Nitrobenzenesulfonyl chloride (39.0 g, 176 mmol) was slowly added to the reaction flask at 0°C. After the addition was complete, the reaction was slowly heated to room temperature and then left overnight for 14-16 hours. The reaction was complete as determined by GC analysis. The reaction was quenched by adding an acetic acid aqueous solution (100 mL, 5 M) to the reaction at 20-25°C. After extraction and liquid separation, the organic phase was washed by adding water (100 mL) and a saturated sodium bicarbonate aqueous solution (60 mL) and concentrated. At 50-55°C, dichloromethane (20 mL) and n-heptane (240 mL) were added, and the mixture was slowly cooled to 20-25°C and stirred at room temperature for 2 hours. After filtration, rinsing with a mixed solvent of dichloromethane/n-heptane, and drying, 39.2 g of the compound represented by formula **(VI-1)** as a solid product was obtained, with an overall yield of 86% over two steps. The ee value of the solid product was 95%, as measured by chiral column HPLC.

MS, *m*/*z* (ESI): 286.0 [M+H]⁺.

¹H NMR, (400 MHz, CDCl₃) δ 8.41 (d, *J =* 8.0 Hz, 2H), 8.15 (d, *J =* 8.0 Hz, 2H), 4.14-4.07 (m, 2H), 2.47 (s, 1H), 2.36 (br, 1H), 1.50 (s, 3H).
The compound represented by formula **(VI-2)**

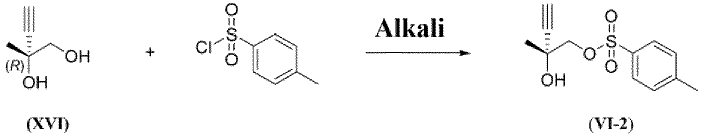

A solution of the compound represented by formula **(XVI)** (15.0 g, 150 mmol) in dichloromethane (90 mL) was added to a reactor, followed by triethylamine (30.3 g, 300 mmol), and the reaction was placed in an ice-water bath at 0-5°C and stirred until dissolved uniform. p-Nitrobenzenesulfonyl chloride (31.4 g, 165 mmol) was slowly added to the reaction flask in batches at 0-5°C and reacted at 0-5°C for 1 hour. Subsequently, the reaction product was slowly heated to room temperature of 20-25°C and reacted under stirring for 14-16 hours. The reaction was complete as determined by GC analysis. The reaction was quenched by adding an acetic acid aqueous solution. The reaction product was concentrated and placed at 0-4°C, whereupon a solid precipitated, which was filtered and dried to obtain 24.5 g of the compound represented by formula **(VI-2)** with a yield of 64.3%. The ee value of the solid product was 97.4% as measured by chiral column HPLC.

MS, *m*/*z* (ESI): 255.1 [M+H]⁺.

¹H NMR (400 MHz, CDCl₃) δ 7.83-7.79 (m, 2H), 7.38-7.31 (m, 2H), 3.98 (q, *J=* 9.7 Hz, 2H), 2.45 (s, 4H), 2.44 (s, 1H), 1.47 (s, 3H).
The compound represented by formula **(VI-3)**

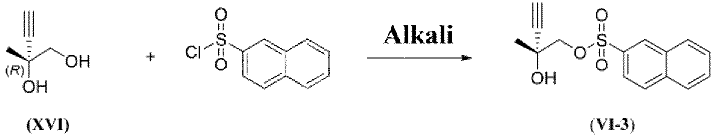

A solution of the compound represented by formula **(XVI)** (25.0 g, 250 mmol) in dichloromethane (150 mL) was added to a reaction flask, followed by 2,6-dimethylpyridine (53.5 g, 500 mmol), and the reaction was placed in an ice-water bath at 0-5°C and stirred until dissolved uniform. 2-Naphthalenesulfonyl chloride (62.3 g, 275 mmol) was slowly added to the reaction flask in batches at 0-5°C. Subsequently, the mixture was slowly heated to room temperature of 20-25°C and reacted under stirring for 14-16 hours. The reaction was complete as determined by GC analysis. The reaction was quenched by adding an acetic acid aqueous solution (150 mL, 5 M) to the reaction at 20-25°C, and after extraction and liquid separation, the organic phase was washed with an acetic acid aqueous solution and water. After concentration, n-heptane was added to precipitate a solid, which was dried to obtain 62.3 g of the compound represented by formula **(VI-3)** as a solid product with a yield of 85.9%. The ee value of the solid product was 97.8%, as measured by chiral column HPLC, which met the quality requirements.

MS, *m*/*z* (ESI): 291.1 [M+H]⁺.

¹H NMR (400 MHz, CDCl₃) δ 8.52 (d, *J =* 1.9 Hz, 1H), 8.01 (dd, *J =* 8.2, 4.7 Hz, 2H), 7.94 (dd, *J =* 8.2, 1.3 Hz, 1H), 7.89 (dd, *J =* 8.7, 1.9 Hz, 1H), 7.68 (dddd, *J* = 19.3, 8.2, 6.9, 1.4 Hz, 2H), 4.05 (q, *J =* 9.8 Hz, 2H), 2.41 (s, 1H), 2.39 (s, 1H), 1.48 (s, 3H).

### Example 9

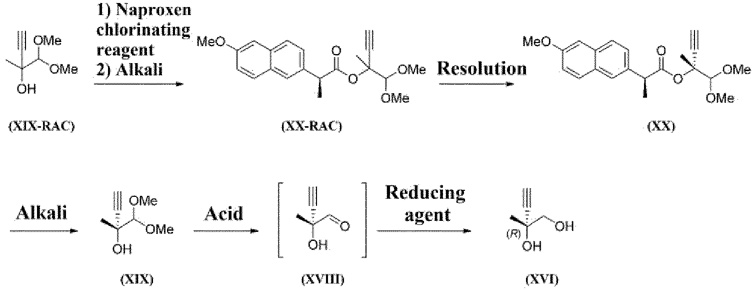

### Step I. Esterification step: Preparation of the compound represented by formula (XX-RAC)

The compound represented by formula **(XIX-RAC)** (72.1 g, 500 mmol), dichloromethane (360 mL), and pyridine (79.1 g, 1.0 mol) were added to a reactor, and the reaction was placed in a water bath at 0°C. A solution of the prepared naproxen chloride (136.8 g, 550 mmol) in dichloromethane (550 mL) was dropwise added to the reaction flask and reacted at 0°C for 4 hours. The reaction was complete as determined by GC analysis. The reaction was quenched by adding an acetic acid aqueous solution, and the reaction product was washed with water and a saturated sodium bicarbonate aqueous solution. After concentration, toluene and n-heptane were added to precipitate a white solid, which was dried to obtain 147.0 g of the compound represented by formula **(XX-RAC).** The yield of this step was 82.5%. The product was a white or off-white solid powder.

MS, *m*/*z* (ESI): 357.2 [M+H]⁺.

¹H NMR (400 MHz, CDCl₃) δ 7.71-7.67 (m, 3H), 7.42-7.40 (m, 1H), 7.15-7.10 (m, 2H), 4.61 (s, 1H), 3.91 (s, 3H), 3.70 (m, 1H), 3.47 (s, 3H), 3.27 (s, 3H), 2.55 (s, 1H), 1.63 (s, 3H), 1.57 (d, *J* = 7.2 Hz, 3H).

### Step II. Crystallization and resolution step: Preparation of the compound represented by formula (XX)

A reaction flask was taken, to which the compound represented by formula **(XX-RAC)** (60.0 g, 168 mmol) and isopropanol (100 mL) were added. The mixture was heated to 55-60°C and stirred until dissolved clear. After slow cooling to 0-5°C, the mixture was maintained at 0-5°C under stirring for 12-16 hours. After filtration at 0-5°C, a solid was collected, and the filter cake was rinsed with n-heptane (50 mL) and dried to obtain 22.5 g of a solid, with a yield of 37.5%. The *dr* value of the solid product was 88.5%, as measured by chiral column HPLC.

MS, *m*/*z* (ESI): 357.2 [M+H]⁺.

¹H NMR (400 MHz, CDCl₃) δ 7.71-7.66 (m, 3H), 7.41-7.38 (m, 1H), 7.15-7.11 (m, 2H), 4.63 (s, 1H), 3.91 (s, 3H), 3.86 (m, 1H), 3.51 (s, 3H), 3.39 (s, 3H), 2.55 (s, 1H), 1.57 (s, 3H), 1.56 (d, *J =* 7.2 Hz, 3H).

### Step III. Ester hydrolysis step: Preparation of the compound represented by formula (XIX)

A reaction flask was taken, to which the compound represented by formula **(XX)** (20.0 g, 56.1 mmol) and a mixed solvent of tetrahydrofuran (100 mL) and water (10 mL) were added and uniformly stirred. Sodium hydroxide solid (4.5 g, 112.3 mmol) was added in batches at 20-25°C and reacted under stirring at 20-25°C for 3 hours. The reaction was complete as determined by HPLC analysis. The reacted mixture was filtered to remove a solid naproxen sodium salt, and the solid filter cake was rinsed with tetrahydrofuran (50 mL). The filtrates were combined. Dichloromethane (100 mL x 2) was added for solvent displacement, and after concentration, a solution of the compound represented by formula **(XIX)** in dichloromethane was obtained, which was directly brought to the next step.

MS, *m*/*z* (ESI): 143.1 [M-H]⁻.

### Step IV. Acetal hydrolysis-reduction step: Preparation of the compound represented by formula (XVI)

At room temperature of 20-25°C, trifluoroacetic acid (50 mL) was added to the solution of the compound represented by formula **(XIX)** in dichloromethane as obtained in the previous step, uniformly stirred, and heated to 43-45°C, and the reaction was stirred for 4-5 hours. The reaction mixture was cooled to room temperature, and water (200 mL) and dichloromethane (200 mL) were added to the mixture. After liquid separation, dichloromethane (100 mL x 2) was added for extraction, and after washing with a saturated sodium bicarbonate aqueous solution (200 mL), followed by concentration, a solution of the compound represented by formula **(XVIII)** in dichloromethane was obtained, which was directly brought to the next step.

MS, *m*/*z* (ESI): 97.1 [M-H]⁻.

At room temperature of 20-25°C, methanol (10 mL) was added to the solution of the compound represented by formula **(XVIII)** in dichloromethane as obtained in the previous step and uniformly stirred, and sodium triacetoxyborohydride (23.7 g, 112 mmol) was slowly added under stirring at 20-25°C. After the addition was complete, the mixture was further stirred for 3-4 hours. The reaction was complete as determined by HPLC analysis. At room temperature, a sodium chloride aqueous solution (100 mL) was added, and after liquid separation, dichloromethane (50 mL x 5) was added for extraction. After concentration, 4.6 g of the compound represented by formula **(XVI)** was obtained, with a yield of 82.0% over three steps of the tandem reaction.

MS, *m*/*z* (ESI): 101.1 [M+H]⁺.

¹H NMR was the same as that reported previously.

## Claims

1. A compound of formula **(VI)** or a stereoisomer and salt thereof, **characterized in that** 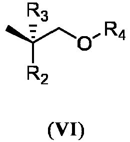
R₂ is selected from hydrogen, deuterium, hydroxyl, amino, C₁₋₃ alkyl, C₁₋₃ deuteroalkyl, C₁₋₃ haloalkyl, C₁₋₃ alkoxy, halo C₁₋₃ alkoxy, or C₁₋₃ hydroxyalkyl, optionally substituted with one or more substituents selected from deuterium, halogen, amino, hydroxyl, cyano, nitro, C₁₋₃ alkyl, C₂₋₄ group, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl;
R₃ is selected from C₁₋₃ alkyl, C₂₋₃ alkenyl, or C₂₋₃ alkynyl, optionally substituted with one or more substituents selected from deuterium, halogen, amino, hydroxyl, cyano, nitro, C₁₋₃ alkyl, C₂₋₄ group, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl;
R₄ is selected from -C(O)(CRₐₐR_{bb})ₙRₐ or -(CRₐₐR_{bb})ₘR_{b};
Rₐₐ or R_{bb} is each independently selected from hydrogen, deuterium, halogen, hydroxyl, amino, or C₁₋₃ alkyl, optionally substituted with one or more substituents selected from deuterium, halogen, amino, hydroxyl, cyano, nitro, C₁₋₃ alkyl, C₂₋₄ group, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl;
Rₐ is selected from C₆₋₁₀ aryl or a 6- to 10-membered heteroaryl containing 1-3 N, O, or S atoms, wherein the C₆₋₁₀ aryl or the 6- to 10-membered heteroaryl containing 1-3 N, O, or S atoms are optionally substituted with one or more substituents selected from deuterium, halogen, amino, hydroxyl, cyano, nitro, C₁₋₃ alkyl, C₂₋₄ group, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl;
R_{b} is selected from hydroxyl protecting groups, preferably substituted or unsubstituted sulfonyl;
m is 0, 1, or 2; and
n is 0, 1, or 2.

2. The compound of formula **(VI)** or the stereoisomer and salt thereof according to claim 1, **characterized in that**
R₂ is selected from hydrogen, deuterium, amino, hydroxyl, cyano, nitro, fluorine, chlorine, bromine, methyl, ethyl, isopropyl, methoxy, ethoxy, propoxy, dimethoxymethyl, dimethoxyethyl, fluoromethyl, fluoroethyl, difluoromethyl, difluoroethyl, trifluoromethyl, or trifluoroethyl;
R₃ is selected from methyl, ethyl, propyl, vinyl, allyl, propenyl, ethynyl, propynyl, or propargyl;
R₄ is selected from -C(O)(CRₐₐR_{bb})ₙRₐ or -(CRₐₐR_{bb})ₘR_{b};
Rₐₐ or R_{bb} is each independently selected from hydrogen, deuterium, fluorine, chlorine, hydroxyl, amino, methyl, ethyl, propyl, or isopropyl;
Rₐ is selected from phenyl, naphthyl, pyridine, pyrimidine, or a six-membered ring fused six-membered heteroaryl containing 1-3 N, O, or S atoms, wherein the phenyl, naphthyl, pyridine, pyrimidine, or six-membered ring fused six-membered heteroaryl containing 1-3 N, O, or S atoms is optionally substituted with one or more substituents selected from hydrogen, deuterium, amino, hydroxyl, cyano, nitro, fluorine, chlorine, bromine, methyl, ethyl, isopropyl, methoxy, ethoxy, fluoromethyl, fluoroethyl, difluoromethyl, difluoroethyl, trifluoromethyl, or trifluoroethyl;
R_{b} is selected from benzenesulfonyl, p-toluenesulfonyl, p-nitrobenzenesulfonyl, p-methoxybenzenesulfonyl, p-trifluoromethylbenzenesulfonyl, p-chlorobenzenesulfonyl, or 2-naphthalenesulfonyl, preferably p-nitrobenzenesulfonyl or 2-naphthalenesulfonyl;
m is 0, 1, or 2; and
n is 0, 1, or 2.

3. The compound of formula **(VI)** or the stereoisomer and salt thereof according to claim 1 or 2, **characterized in that** the compound is further as represented by general formula (XVII):

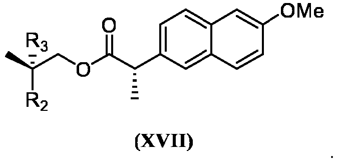

4. A compound of formula (IX) or a stereoisomer and salt thereof, **characterized in that** 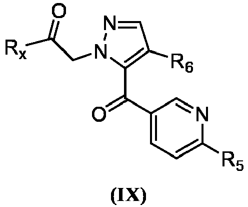
Rₓ is selected from hydrogen, deuterium, C₁₋₃ alkyl, halogen, C₁₋₃ deuteroalkyl, C₁₋₃ haloalkyl, C₁₋₃ alkoxy, halo C₁₋₃ alkoxy, or C₁₋₃ hydroxyalkyl, optionally substituted with one or more substituents selected from deuterium, halogen, amino, hydroxyl, cyano, nitro, C₁₋₃ alkyl, C₂₋₄ group, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl; preferably, Rₓ is selected from hydrogen, deuterium, methyl, ethyl, n-propyl, isopropyl, fluorine, chlorine, or bromine;
R₅ is selected from hydrogen, deuterium, C₁₋₃ alkyl, halogen, C₁₋₃ deuteroalkyl, C₁₋₃ haloalkyl, C₁₋₃ alkoxy, halo C₁₋₃ alkoxy, or C₁₋₃ hydroxyalkyl, optionally substituted with one or more substituents selected from deuterium, halogen, amino, hydroxyl, cyano, nitro, C₁₋₃ alkyl, C₂₋₄ group, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl; preferably, R₅ is selected from hydrogen, deuterium, methyl, ethyl, n-propyl, isopropyl, fluorine, chlorine, or bromine; and
R₆ is selected from hydrogen, deuterium, C₁₋₃ alkyl, C₂₋₃ alkenyl, C₂₋₃ alkynyl halogen, cyano, amino, or hydroxyl, preferably hydrogen, deuterium, methyl, ethyl, n-propyl, isopropyl, fluorine, chlorine, bromine, cyano, vinyl, ethynyl, amino, or hydroxyl.

5. The compound or the stereoisomer and salt thereof according to any one of claims 1-4, **characterized in that** the structure of the compound is as follows:

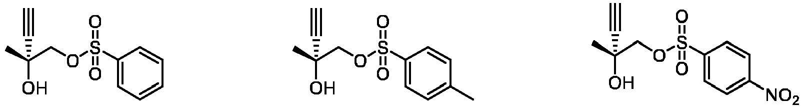

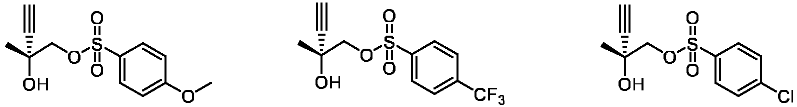

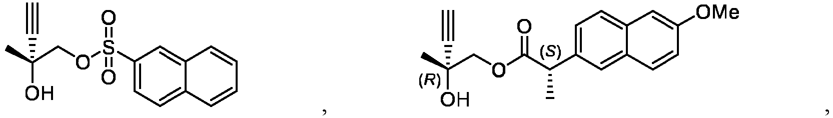

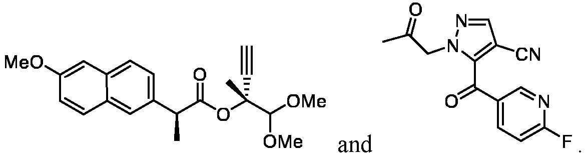

6. The compound of formula (IX) or the stereoisomer and salt thereof according to claim 4, **characterized in that** an intermediate for forming general formula (IX) is as represented by general formula (X): 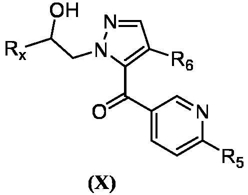 preferably

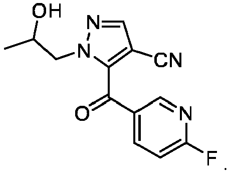

7. The compound of formula (IX) or the stereoisomer and salt thereof according to claim 4, **characterized in that** an intermediate for forming general formula (IX) is as represented by general formula (XI): 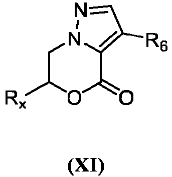 preferably

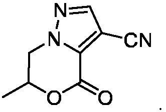

8. A method for preparing the compound of formula (IX) or the stereoisomer and salt thereof according to claim 4, **characterized by** comprising the following step: 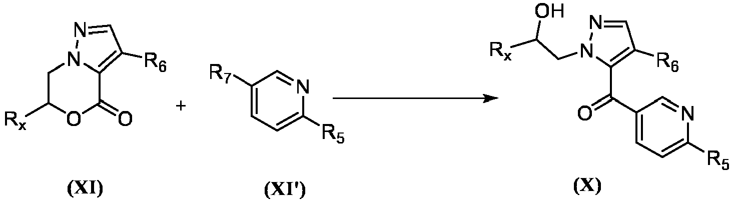 reacting formula **(XI)** with formula **(XI')** to obtain formula **(X),** wherein
Rₓ is selected from hydrogen, deuterium, C₁₋₃ alkyl, or halogen, preferably hydrogen, deuterium, methyl, ethyl, n-propyl, isopropyl, fluorine, chlorine, or bromine;
R₅ is selected from hydrogen, deuterium, C₁₋₃ alkyl, or halogen, preferably hydrogen, deuterium, methyl, ethyl, n-propyl, isopropyl, fluorine, chlorine, or bromine;
R₆ is selected from hydrogen, deuterium, C₁₋₃ alkyl, halogen, cyano, amino, or hydroxyl, preferably hydrogen, deuterium, methyl, ethyl, n-propyl, isopropyl, fluorine, chlorine, bromine, cyano, amino, or hydroxyl;
R₇ is selected from halogen, preferably fluorine, chlorine, or bromine; preferably, the reaction is carried out in the presence of an organometallic reagent that is selected from an organolithium reagent or an organomagnesium reagent, preferably n-butyl lithium, sec-butyl lithium, isobutyl lithium, tert-butyl lithium, isopropyl magnesium chloride, isopropyl magnesium bromide, or isopropyl magnesium chloride-lithium chloride, more preferably n-butyl lithium; and
preferably, a solvent used in the reaction is selected from one or more of toluene, xylene, acetonitrile, 1,4-dioxane, tetrahydrofuran, 2-methyltetrahydrofuran, ethylene glycol dimethyl ether, or methyl tert-butyl ether, preferably a mixed solvent of tetrahydrofuran and toluene or ethylene glycol dimethyl ether and methyl tert-butyl ether, more preferably tetrahydrofuran and toluene at a volume ratio of 4 : 1-1 : 4 or ethylene glycol dimethyl ether and methyl tert-butyl ether at a volume ratio of 1 : 5-1 : 1, further preferably ethylene glycol dimethyl ether and methyl tert-butyl ether at a volume ratio of 1 : 2 or tetrahydrofuran and toluene at a volume ratio of 1.5 : 1.

9. A method for preparing the compound of formula (IX) or the stereoisomer and salt thereof according to claim 4 or 8, **characterized by** comprising the following step: 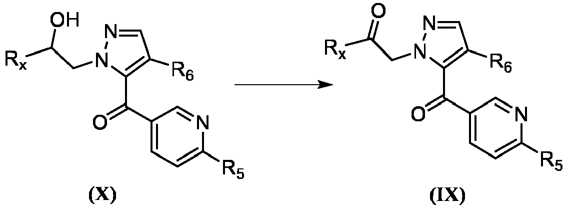
preferably, the reaction is carried out in the presence of an oxidant selected from one or more of sodium hypochlorite, calcium hypochlorite, Dess-Martin periodinane, 2-iodoxybenzoic acid, iodobenzene diacetate, an oxalyl chloride-dimethyl sulfoxide combination reagent, pyridine-sulfur trioxide, 2,2,6,6-tetramethylpiperidine oxide, pyridine-N-oxide, or trichloroisocyanuric acid, preferably a mixed oxidant of 2,2,6,6-tetramethylpiperidine oxide and trichloroisocyanuric acid, more preferably 2,2,6,6-tetramethylpiperidine oxide and trichloroisocyanuric acid at a molar ratio of 1 : 4-1 : 40, further preferably 2,2,6,6-tetramethylpiperidine oxide and trichloroisocyanuric acid at a molar ratio of 1 : 10; and
preferably, the solvent used in the reaction is selected from dichloromethane, chloroform, toluene, chlorobenzene, acetonitrile, 1,4-dioxane, tetrahydrofuran, 2-methyltetrahydrofuran, ethylene glycol dimethyl ether, methyl tert-butyl ether, methanol, N,N-dimethylformamide, N,N-dimethylacetamide, or dimethyl sulfoxide, preferably dichloromethane.

10. A method for preparing the compound of formula (IX) or the stereoisomer and salt thereof according to claim 4, **characterized in that** the preparation of general formula **(V)** comprises the following step: 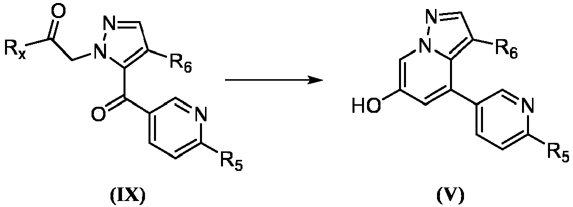
preferably, the reaction is carried out in the presence of an alkali selected from one or more of sodium carbonate, sodium hydroxide, sodium methoxide, sodium ethoxide, sodium isopropoxide, sodium tert-butoxide, sodium hydride, potassium carbonate, potassium hydroxide, potassium methoxide, potassium ethoxide, potassium isopropoxide, potassium tert-butoxide, cesium carbonate, lithium carbonate, lithium hydroxide, lithium chloride, lithium methoxide, lithium ethoxide, lithium isopropoxide, or lithium tert-butoxide, preferably a mixture of lithium hydroxide and lithium chloride, more preferably lithium hydroxide and lithium chloride at a molar ratio of 1 : 4-4 : 1, further preferably lithium hydroxide and lithium chloride at a molar ratio of 1 : 1; and
preferably, a solvent used in the reaction is selected from one or more of toluene, xylene, acetonitrile, 1,4-dioxane, tetrahydrofuran, 2-methyltetrahydrofuran, ethylene glycol dimethyl ether, methyl tert-butyl ether, methanol, ethanol, tert-butanol, water, N,N-dimethylformamide, N,N-dimethylacetamide, or dimethyl sulfoxide, preferably a mixed solvent of ethylene glycol dimethyl ether, methyl tert-butyl ether, and water, more preferably ethylene glycol dimethyl ether, methyl tert-butyl ether, and water at a volume ratio of 5-10 : 30-50 : 1, further preferably ethylene glycol dimethyl ether, methyl tert-butyl ether, and water at a volume ratio of 8 : 40 : 1.

11. A method for preparing the compound of formula **(VI)** or the stereoisomer and salt thereof according to claim 1, **characterized by** comprising the following step: 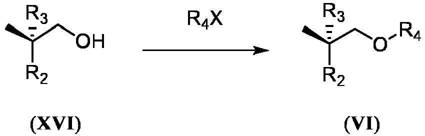
reacting formula **(XVI)** with R₄X to obtain formula **(VI),**
wherein R₂ is selected from hydrogen, deuterium, hydroxyl, amino, or C₁₋₃ alkyl, preferably hydrogen, deuterium, hydroxyl, amino, methyl, ethyl, propyl, or isopropyl;
R₃ is selected from C₁₋₃ alkyl, C₁₋₃ alkenyl, or C₁₋₃ alkynyl, preferably methyl, ethyl, propyl, vinyl, allyl, propenyl, ethynyl, propynyl, or propargyl;
R₄ is selected from benzenesulfonyl, p-toluenesulfonyl, p-nitrobenzenesulfonyl, p-methoxybenzenesulfonyl, p-trifluoromethylbenzenesulfonyl, p-chlorobenzenesulfonyl, or 2-naphthalenesulfonyl, preferably p-nitrobenzenesulfonyl or 2-naphthalenesulfonyl;
X is selected from halogen, preferably fluorine, chlorine, or bromine;
preferably, the alkali is selected from pyridine, 2-methylpyridine, 4-methylpyridine, 2,6-dimethylpyridine, 4-dimethylaminopyridine, imidazole, 1-methylimidazole, triethylamine, diisopropylethylamine, 1,8-diazabicyclo[5.4.0]undec-7-ene, triethylenediamine, sodium carbonate, sodium hydroxide, potassium carbonate, potassium hydroxide, cesium carbonate, lithium carbonate, or lithium hydroxide, preferably 2,6-dimethylpyridine or triethylamine; and
preferably, the solvent used in the reaction is selected from dichloromethane, chloroform, toluene, chlorobenzene, acetonitrile, 1,4-dioxane, tetrahydrofuran, 2-methyltetrahydrofuran, ethylene glycol dimethyl ether, methyl tert-butyl ether, N,N-dimethylformamide, N,N-dimethylacetamide, or dimethyl sulfoxide, preferably dichloromethane.

12. The method for preparing the compound of formula (VI) or the stereoisomer and salt thereof according to claim 11, **characterized in that** the preparation of general formula **(XVI)** comprises the following step: 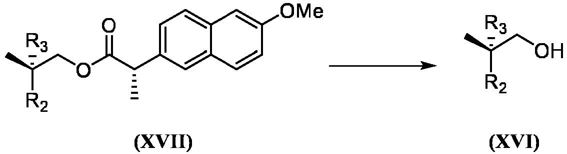
preferably, the reaction is carried out in the presence of an alkali selected from sodium carbonate, sodium hydroxide, sodium methoxide, sodium ethoxide, sodium isopropoxide, sodium tert-butoxide, sodium hydride, potassium carbonate, potassium hydroxide, potassium methoxide, potassium ethoxide, potassium isopropoxide, potassium tert-butoxide, cesium carbonate, lithium carbonate, lithium hydroxide, lithium methoxide, lithium ethoxide, lithium isopropoxide, or lithium tert-butoxide, preferably sodium hydroxide; and
preferably, the solvent used in the reaction is selected from one or more of acetonitrile, 1,4-dioxane, tetrahydrofuran, 2-methyltetrahydrofuran, ethylene glycol dimethyl ether, methyl tert-butyl ether, methanol, ethanol, isopropanol, tert-butanol, ethylene glycol, or water, preferably a mixed solvent of tetrahydrofuran and water, more preferably tetrahydrofuran and water at a volume ratio of 40 : 1-30 : 1, further preferably tetrahydrofuran and water at a volume ratio of 33 : 1.

13. The method for preparing the compound of formula (VI) or the stereoisomer and salt thereof according to claim 12, **characterized in that** the preparation of general formula (XVII) comprises the following step: 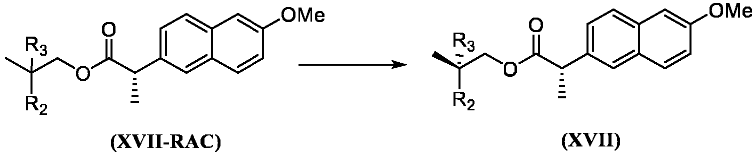 preferably, a resolution solvent is selected from one or more of dichloromethane, chloroform, toluene, chlorobenzene, acetonitrile, acetic acid, 1,4-dioxane, tetrahydrofuran, 2-methyltetrahydrofuran, ethylene glycol dimethyl ether, methyl tert-butyl ether, methanol, N,N-dimethylformamide, N,N-dimethylacetamide, or dimethyl sulfoxide, preferably a combination of toluene and acetonitrile or a combination of toluene and acetic acid, more preferably toluene and acetonitrile solvents used at a volume ratio selected from 20 : 1-3 : 1 or toluene and acetic acid solvents at a volume ratio selected from 20 : 1-5 : 1, further preferably toluene and acetonitrile solvents at a volume ratio selected from 10 : 1-4 : 1 or toluene and acetic acid solvents at a volume ratio selected from 15 : 1-10 : 1, most preferably toluene and acetonitrile solvents at a volume ratio of 4 : 1 or toluene and acetic acid solvents at a volume ratio of 12 : 1.

14. The method for preparing the compound of formula (VI) or the stereoisomer and salt thereof according to claim 13, **characterized in that** the preparation of general formula (XVII-RAC) comprises the following step: 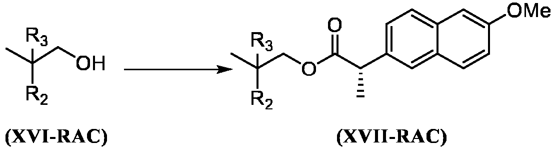
preferably, the reaction is carried out in a naproxen chlorinating reagent selected from oxalyl chloride, thionyl dichloride, phosphorus oxychloride, phosphorus pentachloride, or triphosgene, preferably oxalyl chloride and triphosgene;
preferably, the reaction is carried out in the presence of an alkali selected from pyridine, 2-methylpyridine, 4-methylpyridine, 2,6-dimethylpyridine, 4-dimethylaminopyridine, imidazole, 1-methylimidazole, triethylamine, diisopropylethylamine, 1,8-diazabicyclo[5.4.0]undec-7-ene, triethylenediamine, sodium carbonate, sodium hydroxide, potassium carbonate, potassium hydroxide, cesium carbonate, lithium carbonate, or lithium hydroxide, preferably 2,6-dimethylpyridine; and
preferably, the solvent used in the reaction is selected from dichloromethane, chloroform, toluene, chlorobenzene, acetonitrile, 1,4-dioxane, tetrahydrofuran, 2-methyltetrahydrofuran, ethylene glycol dimethyl ether, methyl tert-butyl ether, N,N-dimethylformamide, N,N-dimethylacetamide, or dimethyl sulfoxide, preferably dichloromethane.
